(19) Europäisches Patentamt
European Patent Office
Office européen des brevets

(11) EP 4 385 527 A1

# (12) EUROPEAN PATENT APPLICATION

(43) Date of publication:
**19.06.2024 Bulletin 2024/25**

(21) Application number: **23213586.3**

(22) Date of filing: **01.12.2023**

(51) International Patent Classification (IPC):
***A61L 2/08*** *(2006.01)* ***A61L 31/04*** *(2006.01)*

(52) Cooperative Patent Classification (CPC):
(C-Sets available)
**A61L 31/049; A61L 2/081; A61L 2/087** (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **12.12.2022 JP 2022198168**

(71) Applicant: **Sumitomo Rubber Industries, Ltd.**
**Kobe-shi, Hyogo 651-0072 (JP)**

(72) Inventors:
- **KIDA, Yogun**
  **Kobe-shi, Hyogo, 651-0072 (JP)**
- **NOJIRI, Kazuki**
  **Kobe-shi, Hyogo, 651-0072 (JP)**
- **TAJIMA, Kei**
  **Kobe-shi, Hyogo, 651-0072 (JP)**
- **SHIBATA, Yusuke**
  **Kobe-shi, Hyogo, 651-0072 (JP)**

(74) Representative: **Manitz Finsterwald**
**Patent- und Rechtsanwaltspartnerschaft mbB**
**Martin-Greif-Strasse 1**
**80336 München (DE)**

## (54) MEDICAL RUBBER PRODUCT

(57) An object of the present invention is to provide a sterilization method for a medical rubber product in which non-eluting characteristics are maintained even after sterilization with gamma ray and with which less troubles occur in a medical product manufacturing process. The medical rubber product according to the present invention is a medical rubber product formed from an elastic material and sterilized by being irradiated with gamma ray or electron ray (beta ray), wherein, when measurement is performed on a surface portion of the elastic material through ATR by using an FT-IR, if an area of an infrared absorption peak at around a wave number of 1650 $cm^{-1}$ in an infrared absorption spectrum obtained through the measurement is defined as As and an area of an infrared absorption peak at around a wave number of 1470 $cm^{-1}$ in the infrared absorption spectrum is defined as Bs, $Ss=(As/Bs)\times 100\leq 6$ is satisfied.

EP 4 385 527 A1

(52) Cooperative Patent Classification (CPC): (Cont.)

C-Sets
**A61L 31/049, C08L 9/10**

## Description

BACKGROUND OF THE INVENTION

Field of the Invention

The present invention relates to a medical rubber product and more specifically relates to a sterilized medical rubber product.

Background Art

Medical rubber plugs for tightly closing openings of syringes, vials, and the like are required to have many characteristics such as non-eluting characteristics, high cleanability, chemical resistance, resistance to needle piercing, self-sealability, and high slidability. Quality characteristics that are required of the medical rubber plugs should, in terms of use of the medical rubber plugs, comply with the regulations stipulated in "Test for Rubber Closure for Aqueous Infusions" of the 17th edition of the Japanese Pharmacopoeia.

For example, Japanese Laid-Open Patent Publication No. H10-179690 discloses a rubber plug for a pharmaceutical agent container, the rubber plug being characterized by being obtained by: blending 5 to 25 parts by weight of fine powder of polyethylene with an ultrahigh molecular weight per 100 parts by weight of a halogenated isobutylene-isoprene rubber; and vulcanizing the resultant halogenated isobutylene-isoprene rubber by using at least one of 2-substituted-4,6-dithiol-s-triazine derivatives or by using an organic peroxide, in the absence of a zinc compound.

There is an increasing demand for medical rubber products (syringe gaskets, vial plugs, and the like) to be delivered in a state of guaranteeing sterilization thereof, i.e., to be ready-to-use (RTU). As methods for guaranteeing sterilization, there are methods involving sterilization with high-pressure steam, sterilization with ethylene oxide gas (EOG), and sterilization with gamma ray. The method involving sterilization with gamma ray has an advantage that a medical rubber product can be sterilized in a state of being packaged and thus can be delivered without opening the package. Meanwhile, the method involving sterilization with EOG has environment-related issues. In view of this, the method for guaranteeing sterilization tends to be switched to the method involving sterilization with gamma ray.

The method involving sterilization with gamma ray guarantees sterilization by means of absorbed dose setting and actually measured values. If a plurality of medical rubber products are put into a packaging bag and sterilization with gamma ray is performed, unevenness among the medical rubber products might occur in the packaging bag. Thus, even when the packaging bag is irradiated with a predetermined radiation dose of gamma ray, variation in the absorbed dose of gamma ray occurs in the packaging bag. This gives rise to: medical rubber products having low absorbed doses of gamma ray; and medical rubber products having high absorbed doses of gamma ray. However, it is necessary to ensure, for each medical rubber product, a minimum absorbed dose with which the medical rubber product can be sterilized. Thus, it is necessary to irradiate the packaging bag with at least the minimum absorbed dose of gamma ray. This gives rise to medical rubber products that absorb an excessive dose of gamma ray at the time of sterilization with gamma ray, in the packaging bag.

Japanese Laid-Open Patent Publication No. 2002-301133 discloses: a rubber composition containing an isobutylene copolymer as a main component and having a density not higher than 0.95, the rubber composition being for use in a medical rubber plug or a medical rubber product on which radiation treatment is easily performed; and a crosslinked product of the rubber composition.

Japanese Laid-Open Patent Publication (Translation of PCT Application) No. 2017-531604 discloses a method for packaging a part (1) made from an elastomer, such as a plug for a pharmaceutical agent container. The method includes: a step of packing the part (1) into a primary bag (10) made from a material substantially impermeable with air; and a step of applying an atmosphere with at least 80% of nitrogen to the inside of the primary bag (10). In the method, the primary bag (10) is put into a secondary bag (20), and the interval between the primary bag (10) and the secondary bag (20) is set to be in a vacuum state.

When a medical rubber product is sterilized by being irradiated with gamma ray, cleavage and crosslinking simultaneously occur in a polymer that forms the medical rubber product. If an excessive dose of gamma ray is absorbed, cleavage of the main chain of the polymer that forms the medical rubber product is promoted, whereby low-molecular-weight components are generated. Consequently, the eluting performance of the medical rubber product having been sterilized with gamma ray deteriorates. In addition, bleed-out, onto a surface of the rubber product, of the low-molecular-weight components resulting from the cleavage occurs, and medical rubber products come into close contact with each other. Consequently, a trouble of clogging in a parts feeder used in a medical product manufacturing process occurs.

It is also conceivable to blend an antioxidant to the medical rubber product in preparation for absorption of an excessive dose of gamma ray. However, addition of an antioxidant leads to concerns that: eluting characteristics decrease; and a medical preparation is adversely influenced.

The present invention has been made in view of the above circumstances, and an object of the present invention is to provide a sterilization method for a medical rubber product in which non-eluting characteristics are maintained even after sterilization with gamma ray.

SUMMARY OF THE INVENTION

A medical rubber product according to the present invention is a medical rubber product formed from an elastic material and sterilized by being irradiated with gamma ray or electron ray, wherein, when measurement is performed on a surface portion of the elastic material through attenuated total reflection (ATR) by using a Fourier transform infrared spectrophotometer (FT-IR), if an area of an infrared absorption peak at around a wave number of 1650 $cm^{-1}$ in an infrared absorption spectrum obtained through the measurement is defined as As and an area of an infrared absorption peak at around a wave number of 1470 $cm^{-1}$ in the infrared absorption spectrum is defined as Bs, $Ss=(As/Bs)\times 100\leq 6$ is satisfied.

The present invention makes it possible to provide a medical rubber product in which non-eluting characteristics are maintained even after sterilization with gamma ray and with which less troubles occur in a medical product manufacturing process.

BRIEF DESCRIPTION OF THE DRAWINGS

FIG. 1 is a diagram for schematically explaining an example of a packaging mode for medical rubber products according to the present invention;

FIG. 2 is a diagram for schematically explaining another example of the packaging mode for the medical rubber products according to the present invention;

FIG. 3 is a chart showing results of FT-IR measurement performed on a medical rubber product according to the present invention;

FIG. 4 is a chart showing results of FT-IR measurement performed on a medical rubber product in a comparative example; and

FIG. 5 is a diagram for schematically explaining an tack test method for the medical rubber product according to the present invention.

DETAILED DESCRIPTION

A medical rubber product according to the present invention is a medical rubber product formed from an elastic material and sterilized by being irradiated with gamma ray or electron ray, wherein, when measurement is performed on a surface portion of the elastic material through attenuated total reflection (ATR) by using a Fourier transform infrared spectrophotometer (FT-IR), if an area of an infrared absorption peak at around a wave number of 1650 $cm^{-1}$ in an infrared absorption spectrum obtained through the measurement is defined as As and an area of an infrared absorption peak at around a wave number of 1470 $cm^{-1}$ in the infrared absorption spectrum is defined as Bs, $Ss=(As/Bs)\times 100\leq 6$ is satisfied.

The medical rubber product according to the present invention is a medical rubber product sterilized by being irradiated with gamma ray or electron ray. An infrared absorption spectrum (vertical axis: absorbance, horizontal axis: wave number) is obtained through measurement on the surface portion of the elastic material that forms the medical rubber product, the measurement being performed through attenuated total reflection (ATR) by using a Fourier transform infrared spectrophotometer (FT-IR).

As the surface portion of the medical rubber product, a measurement sample is made by being cut out from the surface of the rubber product with use of a razor so as to have a thickness (for example, 0.5 mm to 2.0 mm) that allows the measurement sample to be placed on a measurement device. The measurement sample is placed on the measurement device, and a region, of a surface portion of the measurement sample, having an outer diameter of 1 mm is irradiated with light and is subjected to measurement.

In the infrared absorption spectrum, an infrared absorption peak A having an absorption peak of absorbance at around a wave number of 1650 $cm^{-1}$ is present. The infrared absorption peak corresponds to a carbonyl (C=O stretching vibration) group (a ketone, an aldehyde, a carboxylic acid, an ester, or the like) attributed to an oxide of a polymer composing the elastic material. The phrase "at around a wave number of 1650 $cm^{-1}$" preferably means a region ranging over wave numbers of 1650 $cm^{-1}\pm 100$ $cm^{-1}$ and more preferably means a region ranging over wave numbers of 1650 $cm^{-1}\pm 50$ $cm^{-1}$.

In the infrared absorption spectrum, an infrared absorption peak B having an absorption peak of absorbance at around a wave number of 1470 $cm^{-1}$ is present. The infrared absorption peak B is attributed to scissoring vibration (in-plane deformation vibration) of $-CH_2-$in the main chain of the polymer composing the elastic material. The phrase "at

around a wave number of 1470 $cm^{-1}$" preferably means a region ranging over wave numbers of 1470 $cm^{-1} \pm 50$ $cm^{-1}$ and more preferably means a region ranging over wave numbers of 1470 $cm^{-1} \pm 20$ $cm^{-1}$.

The surface portion of the elastic material that forms the medical rubber product according to the present invention is as follows. That is, if the area of the infrared absorption peak A is defined as As and the area of the infrared absorption peak B is defined as Bs, a value Ss calculated according to Ss=(As/Bs)×100 is preferably not larger than 6, preferably not larger than 5.5, or preferably not larger than 5.0. If the value Ss is not larger than 6, degradation of the polymer composing the elastic material of the medical rubber product is suppressed, and the medical rubber product comes to have excellent non-eluting characteristics. Meanwhile, since the surface portion of the elastic material is oxidized during manufacturing and processing of the medical rubber product, the value Ss is generally larger than 3.0 and more generally not smaller than 3.5.

Cut-out from a center portion of the medical rubber product is performed and measurement is performed on an exposed inside of the medical rubber product through attenuated total reflection (ATR) by using the Fourier transform infrared spectrophotometer (FT-IR), whereby an infrared absorption spectrum (vertical axis: absorbance, horizontal axis: wave number) is obtained.

Specifically, the medical rubber product is cut in the longitudinal direction through the center thereof in a plan view. A measurement sample is obtained by being cut out from a center portion of the obtained cross section with use of the razor so as to have a thickness (for example, 0.5 mm to 2.0 mm) that allows the measurement sample to be placed on the measurement device. The measurement sample is placed on the measurement device, and a region, of a surface portion of the measurement sample (the center portion of the cross section), having an outer diameter of 1 mm is irradiated with light and is subjected to measurement.

In the infrared absorption spectrum, an infrared absorption peak A having an absorption peak of absorbance at around a wave number of 1650 $cm^{-1}$ is present. The infrared absorption peak is attributed to the oxide of the polymer composing the elastic material. The phrase "at around a wave number of 1650 $cm^{-1}$" preferably means a region ranging over wave numbers of 1650 $cm^{-1} \pm 100$ $cm^{-1}$ and more preferably means a region ranging over wave numbers of 1650 $cm^{-1} \pm 50$ $cm^{-1}$.

In the infrared absorption spectrum, an infrared absorption peak B having an absorption peak of absorbance at around a wave number of 1470 $cm^{-1}$ is present. The infrared absorption peak B is attributed to scissoring vibration (in-plane deformation vibration) of $-CH_2$-in the main chain of the polymer composing the elastic material. The phrase "at around a wave number of 1470 $cm^{-1}$" preferably means a region ranging over wave numbers of 1470 $cm^{-1} \pm 50$ $cm^{-1}$ and more preferably means a region ranging over wave numbers of 1470 $cm^{-1} \pm 20$ $cm^{-1}$.

The inside of the elastic material that forms the medical rubber product according to the present invention is as follows. That is, if the area of the infrared absorption peak A is defined as Ai, the area of the infrared absorption peak B is defined as Bi, and Si=(Ai/Bi)×100 is satisfied, a value calculated according to P=(Ss/Si)×100 is preferably not larger than 150, preferably not larger than 140, or preferably not larger than 130. The value P (=(Ss/Si)×100) is an index of the extent of degradation at the inside and the surface portion of the medical rubber product. If the value P is not larger than 150, the extent of degradation at the surface portion is small, and non-eluting characteristics and non-adhesiveness are favorable. The lower limit of the value P is not particularly limited.

[Medical Rubber Product]

The medical rubber product according to the present invention is formed from an elastic material. The elastic material is not particularly limited as long as the elastic material contains a polymer having a methylene chain ($-CH_2-$) in the main chain thereof. The elastic material is preferably composed of a rubber component.

The elastic material is preferably a cured product of a medical rubber composition containing: a (a) base polymer containing a halogenated isobutylene-isoprene rubber; and a triazine derivative as a (c) crosslinking agent. The elastic material is more preferably a cured product of a medical rubber composition containing: a (a) base polymer containing a halogenated isobutylene-isoprene rubber; a (b) polyethylene; and a triazine derivative as a (c) crosslinking agent. Hereinafter, raw materials contained in the medical rubber composition according to the present invention will be described.

First, the (a) base polymer containing a halogenated isobutylene-isoprene rubber will be described. Examples of the halogenated isobutylene-isoprene rubber include: chlorinated isobutylene-isoprene rubber; brominated isobutylene-isoprene rubber; a bromide of a copolymer rubber of isobutylene and p-methylstyrene (brominated isobutylene-para-methylstyrene copolymer rubber); and the like.

As the halogenated isobutylene-isoprene rubber, chlorinated isobutylene-isoprene rubber or brominated isobutylene-isoprene rubber is preferable. The chlorinated isobutylene-isoprene rubber or the brominated isobutylene-isoprene rubber is obtained by, for example, causing a reaction in which: chlorine or bromine is added to an isoprene structural moiety (specifically, a double bond and/or a carbon atom adjacent to the double bond) in an isobutylene-isoprene rubber; or the isoprene structural moiety is substituted with chlorine or bromine. The isobutylene-isoprene

rubber is a copolymer obtained by polymerizing isobutylene and a small amount of isoprene.

The halogen content of the halogenated isobutylene-isoprene rubber is preferably not lower than 0.5% by mass, more preferably not lower than 1% by mass, and further preferably not lower than 1.5% by mass. Meanwhile, the halogen content is preferably not higher than 5% by mass, more preferably not higher than 4% by mass, and further preferably not higher than 3% by mass.

Specific examples of the chlorinated isobutylene-isoprene rubber include at least one of: CHLOROBUTYL 1066 [stabilizer: NS, halogen content: 1.26%, Mooney viscosity: 38 $ML_{1+8}$ (125°C), specific gravity: 0.92] manufactured by JAPAN BUTYL Co., Ltd.; LANXESS X_BUTYL CB1240 manufactured by LANXESS; and the like.

Specific examples of the brominated isobutylene-isoprene rubber include at least one of: BROMOBUTYL 2255 [stabilizer: NS, halogen content: 2.0%, Mooney viscosity: 46 $ML_{1+8}$ (125°C), specific gravity: 0.93] manufactured by JAPAN BUTYL Co., Ltd.; LANXESS X_BUTYL BBX2 manufactured by LANXESS; and the like.

The (a) base polymer may contain a rubber component other than the halogenated isobutylene-isoprene rubber.

Examples of the other rubber component include butyl-based rubbers, isoprene rubber, butadiene rubber, styrene-butadiene rubber, natural rubber, chloroprene rubber, nitrile-based rubbers such as acrylonitrile-butadiene rubber, hydrogenated nitrile-based rubbers, norbornene rubber, ethylene-propylene rubber, ethylene-propylene-diene rubber, acrylic rubber, ethylene-acrylate rubber, fluororubber, chlorosulfonated polyethylene rubber, epichlorohydrin rubber, silicone rubber, urethane rubber, polysulfide rubber, phosphazene rubber, 1,2-polybutadiene, and the like. These rubber components may be used singly, or two or more of these rubber components may be used in combination.

In the case of using the other rubber component, the proportion of the halogenated isobutylene-isoprene rubber contained in the (a) base polymer is preferably not lower than 90% by mass, more preferably not lower than 95% by mass, and further preferably not lower than 98% by mass. A mode in which the (a) base polymer contains only the halogenated isobutylene-isoprene rubber is also preferable.

The medical rubber composition according to the present invention preferably contains the (b) polyethylene. The polyethylene is more likely to absorb gamma ray than the (a) base polymer is. Thus, the polyethylene has an effect of preventing cleavage of a chain of the (a) base polymer due to irradiation with gamma ray. In addition, a polyethylene having a low degree of crystallinity has a branched chain, and it is considered that crosslinking progresses without cleavage of the main chain even upon irradiation with gamma ray. As a result, the eluting performance of the medical rubber composition is considered to be improved.

From this viewpoint, examples of the (b) polyethylene to be used in the present invention can include high-density polyethylene (HDPE) and low-density polyethylene (LDPE). The high-density polyethylene (HDPE) and the low-density polyethylene (LDPE) may be used singly, or the high-density polyethylene (HDPE) and the low-density polyethylene (LDPE) may be used in combination.

In the case of using the high-density polyethylene (HDPE) and the low-density polyethylene (LDPE) in combination, the mass ratio (HDPE/LDPE) of the high-density polyethylene (HDPE) to the low-density polyethylene (LDPE) is preferably not lower than 0.3, more preferably not lower than 0.5, and further preferably not lower than 1.0. Meanwhile, the mass ratio (HDPE/LDPE) is preferably not higher than 5.0, more preferably not higher than 4.0, and further preferably not higher than 3.0. This is because, if the mass ratio (HDPE/LDPE) of the high-density polyethylene (HDPE) to the low-density polyethylene (LDPE) falls within the aforementioned range, a radical absorption effect at the time of irradiation with gamma ray and an appropriate hardness of the rubber can be ensured.

The (b) polyethylene preferably contains a polyethylene having a degree of crystallinity not higher than 70%.

The degree of crystallinity of the high-density polyethylene (HDPE) is preferably 60% to 80%, more preferably 60% to 75%, and further preferably 60% to 70%. The degree of crystallinity of the low-density polyethylene (LDPE) is preferably 30% to 50%, more preferably 30% to 45%, and further preferably 30% to 40%. This is because, if the degree of crystallinity of each polyethylene falls within the aforementioned corresponding range, radicals generated through irradiation with gamma ray are effectively absorbed, and cleavage of the main chain of the polymer is prevented.

The degree of crystallinity of the (b) polyethylene is determined according to the following expression.

$$\text{Degree of crystallinity (\%)} = (\text{measured melting heat quantity (J/g)}/\text{perfect crystal melting heat quantity (J/g)}) \times 100$$

The perfect crystal melting heat quantity (J/g) is 293 J/g (a value in a literature) and is a melting heat quantity of the polyethylene at 100% crystallinity. A measurement method for the melting heat quantity of the polyethylene will be described later.

As the (b) polyethylene, the low-density polyethylene is preferable. The density (g/cm$^3$) of the high-density polyethylene is preferably 0.930 to 0.960 and more preferably 0.930 to 0.950. The density (g/cm$^3$) of the low-density polyethylene is not particularly limited, but is preferably 0.910 to 0.925 and more preferably 0.910 to 0.920.

As the (b) polyethylene, a polyethylene in the form of fine powder is preferably used. The volume-average particle diameter of the polyethylene in the form of fine powder is preferably not smaller than 10 μm, more preferably not smaller than 15 μm, and further preferably not smaller than 20 μm. Meanwhile, the volume-average particle diameter is preferably not larger than 200 μm, more preferably not larger than 160 μm, and further preferably not larger than 120 μm. This is because, if the particle diameter of the polyethylene in the form of fine powder falls within the aforementioned range, it becomes easy for the polyethylene to be evenly mixed or dispersed in the polymer.

The blending amount of the (b) polyethylene per 100 parts by mass of the (a) base polymer is preferably not smaller than 3 parts by mass, more preferably not smaller than 5 parts by mass, and further preferably not smaller than 10 parts by mass. Meanwhile, the blending amount is preferably not larger than 30 parts by mass, more preferably not larger than 25 parts by mass, and further preferably not larger than 20 parts by mass. This is because, if the blending amount of the (b) polyethylene falls within the aforementioned range, radicals generated at the time of irradiation with gamma ray can be effectively absorbed, and cleavage of the main chain of the polymer can be prevented.

The medical rubber composition according to the present invention preferably contains a triazine derivative as the (c) crosslinking agent.

The triazine derivative acts as a crosslinking agent on the halogenated isobutylene-isoprene rubber. Examples of the triazine derivative include a compound represented by a general formula (1).

[Chem. 1]

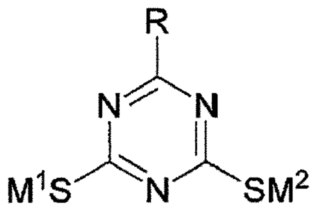

(1)

[in the formula, R represents -SH, $-OR^1$, $-SR^2$, $-NHR^3$, or $-NR^4R^5$ ($R^1$, $R^2$, $R^3$, $R^4$, and $R^5$ each represent an alkyl group, an alkenyl group, an aryl group, an aralkyl group, an alkylaryl group, or a cycloalkyl group. $R^4$ and $R^5$ may be identical to each other or different from each other.). $M^1$ and $M^2$ each represent H, Na, Li, K, 1/2Mg, 1/2Ba, 1/2Ca, an aliphatic primary, secondary, or tertiary amine, a quaternary ammonium salt, or a phosphonium salt. $M^1$ and $M^2$ may be identical to each other or different from each other.]

In the general formula (1), examples of the alkyl group include alkyl groups having 1 to 12 carbon atoms, such as a methyl group, an ethyl group, an n-propyl group, an isopropyl group, an n-butyl group, an isobutyl group, a tert-butyl group, an n-pentyl group, an isopentyl group, a tert-pentyl group, an n-hexyl group, a 1,1-dimethylpropyl group, an octyl group, an isooctyl group, a 2-ethylhexyl group, a decyl group, and a dodecyl group. Examples of the alkenyl group include alkenyl groups having 1 to 12 carbon atoms, such as a vinyl group, an allyl group, a 1-propenyl group, an isopropenyl group, a 2-butenyl group, a 1,3-butadienyl group, and a 2-pentenyl group. Examples of the aryl group include monocyclic aromatic hydrocarbon groups and condensed polycyclic aromatic hydrocarbon groups, and examples thereof include: aryl groups having 6 to 14 carbon atoms, such as a phenyl group, a naphthyl group, an anthryl group, a phenanthryl group, and an acenaphthylenyl group; and the like. Examples of the aralkyl group include aralkyl groups having 7 to 19 carbon atoms, such as a benzyl group, a phenethyl group, a diphenylmethyl group, a 1-naphthylmethyl group, a 2-naphthylmethyl group, a 2,2-diphenylethyl group, a 3-phenylpropyl group, a 4-phenylbutyl group, a 5-phenylpentyl group, a 2-biphenylylmethyl group, a 3-biphenylylmethyl group, and a 4-biphenylylmethyl group. Examples of the alkylaryl group include alkylaryl groups having 7 to 19 carbon atoms, such as a tolyl group, a xylyl group, and an octylphenyl group. Examples of the cycloalkyl group include: cycloalkyl groups having 3 to 9 carbon atoms, such as a cyclopropyl group, a cyclobutyl group, a cyclopentyl group, a cyclohexyl group, a cycloheptyl group, a cyclooctyl group, and a cyclononyl group; and the like.

Specific examples of the triazine derivative represented by the general formula (1) include 2,4,6-trimercapto-s-triazine, 2-methylamino-4,6-dimercapto-s-triazine, 2-(n-butylamino)-4,6-dimercapto-s-triazine, 2-octylamino-4,6-dimercapto-s-triazine, 2-propylamino-4,6-dimercapto-s-triazine, 2-diallylamino-4,6-dimercapto-s-triazine, 2-dimethylamino-4,6-dimercapto-s-triazine, 2-dibutylamino-4,6-dimercapto-s-triazine, 2-di(iso-butylamino)-4,6-dimercapto-s-triazine, 2-dipropylamino-4,6-dimercapto-s-triazine, 2-di(2-ethylhexyl)amino-4,6-dimercapto-s-triazine, 2-dioleylamino-4,6-dimercapto-s-triazine, 2-laurylamino-4,6-dimercapto-s-triazine, 2-anilino-4,6-dimercapto-s-triazine, and sodium salts and disodium salts of these triazine derivatives.

Among these triazine derivatives, 2,4,6-trimercapto-s-triazine, 2-dialkylamino-4,6-dimercapto-s-triazine, and 2-anilino-4,6-dimercapto-s-triazine are preferable, and 2-dibutylamino-4,6-dimercapto-s-triazine is particularly preferable

since 2-dibutylamino-4,6-dimercapto-s-triazine is easy to obtain.

Other examples of the triazine derivative include one or more of 6-[bis(2-ethylhexyl)amino]-1,3,5-triazine-2,4-dithiol, 6-diisobutylamino-1,3,5-triazine-2,4-dithiol, 6-dibutylamino-1,3,5-triazine-2,4-dithiol, 6-dibutylamino-1,3,5-triazine-2,4-dithiol monosodium, 6-anilino-1,3,5-triazine-2,4-dithiol, 1,3,5-triazine-2,4,6-trithiol, and the like.

In the present invention, these triazine derivatives may be used singly, or two or more of these triazine derivatives may be used in combination.

In the medical rubber composition according to the present invention, the amount of the (c) triazine derivative contained per 100 parts by mass of the (a) base polymer component is preferably not smaller than 0.1 parts by mass, more preferably not smaller than 0.3 parts by mass, and further preferably not smaller than 0.5 parts by mass. Meanwhile, the amount is preferably not larger than 2.0 parts by mass, more preferably not larger than 1.4 parts by mass, and further preferably not larger than 1.2 parts by mass. This is because, if the amount of the (c) triazine derivative contained falls within the aforementioned range, a rubber having favorable rubber physical properties (hardness, tensile properties, Cset) and good eluting performance and processability (less susceptibility to scorching) can be obtained.

The medical rubber composition according to the present invention preferably contains no vulcanization accelerator. This is because a vulcanization accelerator could remain in a rubber product obtained as a final product and could elute into a drug solution inside a syringe or a vial. Examples of the vulcanization accelerator include guanidine-based accelerators (e.g., diphenylguanidine), thiuram-based accelerators (e.g., tetramethylthiuram disulfide and tetramethylthiuram monosulfide), dithiocarbamate-based accelerators (e.g., zinc dimethyldithiocarbamate), thiazole-based accelerators (e.g., 2-mercaptobenzothiazole and dibenzothiazyl disulfide), and sulfenamide-based accelerators (N-cyclohexyl-2-benzothiazole sulfenamide and N-t-butyl-2-benzothiazole sulfenamide).

The medical rubber composition according to the present invention may contain a hydrotalcite. The hydrotalcite functions as an anti-scorching agent upon crosslinking in the halogenated isobutylene-isoprene rubber and also has a function of preventing increase in compression set in the medical rubber product. Further, the hydrotalcite functions also as an acid acceptor for absorbing chlorine-based gas and bromine-based gas, which have been generated upon crosslinking in the halogenated isobutylene-isoprene rubber, and preventing occurrence of, for example, crosslinking inhibition due to these gases. Magnesium oxide can also function as an acid acceptor.

Examples of the hydrotalcite include one or more of Mg-Al-based hydrotalcites such as $Mg_{4.5}Al_2(OH)_{13}CO_3 \cdot 3.5H_2O$, $Mg_{4.5}Al_2(OH)_{13}CO_3$, $Mg_4Al_2(OH)_{12}CO_3 \cdot 3.5H_2O$, $Mg_6Al_2(OH)_{16}CO_3 \cdot 4H_2O$, $Mg_5Al_2(OH)_{14}CO_3 \cdot 4H_2O$, and $Mg_3Al_2(OH)_{10}CO_3 \cdot 1.7H_2O$, and the like.

Specific examples of the hydrotalcite include DHT-4A (registered trademark)-2 manufactured by Kyowa Chemical Industry Co., Ltd., and the like.

In the case where the hydrotalcite is used as an acid acceptor in the medical rubber composition according to the present invention, the hydrotalcite is preferably used in combination with MgO. In this case, the blending amount of the hydrotalcite is preferably considered in terms of the total amount of the acid acceptors (hydrotalcite and MgO). The total amount of the acid acceptors (hydrotalcite and MgO) contained per 100 parts by mass of the (a) base polymer component is preferably not smaller than 0.5 parts by mass and more preferably not smaller than 1 part by mass. Meanwhile, the total amount is preferably not larger than 15 parts by mass and more preferably not larger than 10 parts by mass. This is because, if the total amount of the acid acceptors (hydrotalcite and MgO) falls within the aforementioned range, generation of rust on a mold or the like can be suppressed, and defects that raw materials themselves turn into an unwanted object in the form of a white spot can be reduced.

The medical rubber composition according to the present invention may contain a co-crosslinking agent. The co-crosslinking agent is preferably a polyfunctional (meth)acrylate compound. The polyfunctional (meth)acrylate compound is more preferably a difunctional or higher-functional (meth)acrylate-based compound and further preferably a trifunctional or higher-functional (meth)acrylate-based compound. Meanwhile, the polyfunctional (meth)acrylate compound is preferably an octafunctional or lower-functional (meth)acrylate-based compound and more preferably a hexafunctional or lower-functional (meth)acrylate-based compound. Examples of the difunctional or higher-functional (meth)acrylate compound can include a compound having at least two acryloyl groups and/or methacryloyl groups. The term "(meth)acrylate" means "acrylate" and/or "methacrylate".

Examples of the difunctional or higher-functional (meth)acrylate-based compound can include di(meth)acrylate of polyethylene glycol, 1,4-butanediol di(meth)acrylate, 1,6-hexanediol di(meth)acrylate, 1,9-nonanediol di(meth)acrylate, trimethylolpropane tri(meth)acrylate, trimethylolethane tri(meth)acrylate, pentaerythritol tri(meth)acrylate, pentaerythritol tetra(meth)acrylate, glycerin tri(meth)acrylate, dipentaerythritol tri(meth)acrylate, dipentaerythritol tetra(meth)acrylate, dipentaerythritol penta(meth)acrylate, dipentaerythritol hexa(meth)acrylate, tripentaerythritol tetra(meth)acrylate, tripentaerythritol penta(meth)acrylate, tripentaerythritol hexa(meth)acrylate, tripentaerythritol hepta(meth)acrylate, and the like. These co-crosslinking agents may be used singly, or two or more of these co-crosslinking agents may be used in combination.

In the medical rubber composition according to the present invention, a (d) filler may be blended. Examples of the (d) filler include inorganic fillers such as silica, clay, and talc. The filler is further preferably clay or talc. The filler has

a function of adjusting the rubber hardness of the medical rubber product and functions also as an extender for reducing manufacturing cost for the medical rubber product.

Examples of the clay can include calcined clay and kaolin clay. Specific examples of the clay include SILLITIN (registered trademark) Z manufactured by Hoffmann Mineral GmbH, SATINTONE (registered trademark) W manufactured by Engelhard Corporation, NN Kaolin Clay manufactured by Tsuchiya Kaolin Industry Co., Ltd., PoleStar200R manufactured by Imerys Specialties Japan Co., Ltd., and the like.

Specific examples of the talc include High toron A manufactured by Takehara Kagaku Kogyo Co., Ltd., MICRO ACE (registered trademark) K-1 manufactured by Nippon Talc Co., Ltd., MISTRON (registered trademark) Vapor manufactured by Imerys Specialties Japan Co., Ltd., and the like.

In the medical rubber composition according to the present invention, a colorant such as titanium oxide or carbon black, polyethylene glycol as a processing aid or as a crosslinking activator, a plasticizer (for example, paraffin oil), and the like may further be blended in appropriate proportions.

The medical rubber composition according to the present invention is obtained by kneading the (a) base polymer containing the halogenated isobutylene-isoprene rubber, the (b) polyethylene, the triazine derivative as the (c) crosslinking agent, and other blending materials to be added as necessary. The kneading can be performed by using, for example, an open roll, a sealed-type kneader, or the like. The kneaded product is preferably molded in the shape of a ribbon, the shape of a sheet, the shape of a pellet, or the like, and is more preferably molded in the shape of a sheet.

If the kneaded product having the shape of a ribbon, the shape of a sheet, or the shape of a pellet is press-molded, a medical rubber product having a desired shape is obtained. A crosslinking reaction in the medical rubber composition progresses during the pressing. The temperature in the molding is, for example, preferably not lower than 130°C and more preferably not lower than 140°C. Meanwhile, the temperature is preferably not higher than 200°C and more preferably not higher than 190°C. The time for the molding is preferably not shorter than 2 minutes and more preferably not shorter than 3 minutes. Meanwhile, the time is preferably not longer than 60 minutes and more preferably not longer than 30 minutes. The pressure for the molding is preferably not lower than 0.1 MPa and more preferably not lower than 0.2 MPa. Meanwhile, the pressure is preferably not higher than 10 MPa and more preferably not higher than 8 MPa.

Unnecessary portions are cut off and removed from the molded product after the press-molding, such that the molded product has a predetermined shape. The obtained molded product is washed, dried, and packaged to manufacture the medical rubber product.

The JIS-A hardness of the elastic material that forms the medical rubber product according to the present invention is preferably not lower than 30, more preferably not lower than 35, and further preferably not lower than 40. Meanwhile, the JIS-A hardness is preferably not higher than 70, more preferably not higher than 65, and further preferably not higher than 60. This is because, if the JIS-A hardness of the elastic material falls within the aforementioned range, both product performances such as sealing performance and resistance to needle piercing which are required of rubber plugs can be achieved.

The elastic material that forms the medical rubber product according to the present invention has a compression set measured under conditions of 70°C, 22 hours, and 25% according to JIS K 6262. The compression set is preferably not higher than 20%, more preferably not higher than 15%, and further preferably not higher than 10%. This is because, if the compression set of the elastic material falls within the aforementioned range, sealing performance required of rubber plugs is achieved, and a needle hole is swiftly closed at the time of needle removal after needle piercing, whereby no liquid leakage occurs.

[Sterilization Method]

The medical rubber product according to the present invention is preferably sterilized by being irradiated with a radioactive ray and is more preferably sterilized by being irradiated with gamma ray or electron ray. A method for the sterilization preferably includes irradiating a packaging article for the medical rubber product with a radioactive ray, the packaging article accommodating a plurality of the medical rubber products.

Examples of the radioactive ray to be used for the sterilization include $\alpha$-ray (the atomic nucleus of helium), $\beta$-ray (electron ray), and $\gamma$-ray (gamma ray). $\beta$-ray (electron ray) has a very high dose rate (several tens of thousands of times the dose rate of $\gamma$-ray), and thus the sterilization time is short. However, since $\beta$-ray is a particle beam, the penetrating power thereof is small. Meanwhile, gamma ray has a large penetrating power but has a lower dose rate than electron ray, and thus the processing time is elongated. In the present invention, the packaging article, for the medical rubber product, accommodating a plurality of the medical rubber products is preferably sterilized with gamma ray from the viewpoint of sterilizing the packaging article.

Examples of the gamma ray can include gamma rays emitted from cobalt-60, cesium-137, and the like. Gamma ray emitted from cobalt-60 is suitable.

Regarding irradiation with the gamma ray, an absorbed dose of the gamma ray by the medical rubber product

is set through an actual sterilization validation procedure. For ordinary medical devices, operation is performed with a minimum absorbed dose being set to 15 kGy in many cases. A gamma ray radiation dose at which the absorbed doses of the gamma ray by all of the medical rubber products in the packaging article take values not lower than 15 kGy, varies depending on the number of the medical rubber products in the packaging article, how the medical rubber products are present in the packaging article, and the like. In general, irradiation is performed in a dose that falls within a range of not lower than 1.4 times 15 kGy and not higher than 2.0 times 15 kGy. Likewise, if the minimum absorbed dose is set to 20 kGy, irradiation is performed in a dose that falls within a range of not lower than 1.4 times 20 kGy and not higher than 2.0 times 20 kGy, and, if the minimum absorbed dose is set to 25 kGy, irradiation is performed in a dose that falls within a range of not lower than 1.4 times 25 kGy and not higher than 2.0 times 25 kGy. The absorbed dose of the gamma ray can be ascertained through attachment of a dosemeter to an object that is to be irradiated.

The packaging article accommodating the medical rubber products not having yet been irradiated with the gamma ray has an oxygen concentration that is preferably not higher than 5%, more preferably not higher than 3%, and further preferably not higher than 1%. This is because, if the oxygen concentration in the packaging article is set to be not higher than 5%, degradation of each medical rubber product due to irradiation with the gamma ray can be suppressed.

Examples of a method for setting the oxygen concentration in the packaging article to be not higher than 5% can include: a method in which air in the packaging article is substituted with an inert gas; and a method in which an oxygen adsorber is accommodated in the packaging article.

Examples of the inert gas can include: rare gases such as helium gas, neon gas, and argon gas; nitrogen gas; and the like.

Examples of the oxygen adsorber can include AGELESS (commercially available product) which is an iron-based oxygen adsorber, and the like.

The packaging article for accommodating the medical rubber product is not particularly limited as long as the packaging article can be irradiated with the gamma ray. Examples of the form of the packaging article can include the forms of a bag, a box, and the like. Examples of the packaging bag can include packaging bags formed of aluminum or a thermoplastic resin film made from polyethylene, polyamide, or polyester. The packaging bag is preferably one that can be sealed. The packaging box is not particularly limited, and examples of the packaging box can include a box made from paper, a box made from cardboard, and the like.

Examples of the packaging article can include: a packaging article having gas permeability; and a packaging article having non-gas permeability (gas sealability). It is also preferable to use these packaging articles in combination.

Irradiation of the medical rubber product with the gamma ray may be performed on, for example, a packaging article (for example, a cardboard box) further accommodating a plurality of primary packaging articles (for example, packaging bags) accommodating a plurality of the medical rubber products.

FIG. 1 is a diagram for schematically explaining an example of a packaging mode for irradiation with the gamma ray. In the mode shown in FIG. 1, a primary packaging article 3 accommodating a plurality of medical rubber products 1 is further accommodated in a secondary static charge prevention packaging article 5 and a tertiary static charge prevention packaging article 7. As the primary packaging article 3, a packaging article having gas permeability is preferable. As the secondary static charge prevention packaging article 5 and the tertiary static charge prevention packaging article 7, packaging articles capable of sealing gas therein are preferable. Each of the secondary static charge prevention packaging article 5 and the tertiary static charge prevention packaging article 7 is preferably sealed with a heat seal 9. In the case of using an oxygen adsorber 11, the oxygen adsorber 11 is preferably disposed between the primary packaging article 3 and the secondary packaging article 5 such that the oxygen adsorber 11 does not come into direct contact with the medical rubber products 1. By disposing the oxygen adsorber 11 in the secondary packaging article 5, the oxygen concentration in each of the secondary packaging article 5 and the primary packaging article 3 can be set to be not higher than 5%. Irradiation with the gamma ray can be performed with a plurality of the tertiary static charge prevention packaging articles 7 being accommodated in a quaternary packaging article (for example, a cardboard box).

FIG. 2 is a diagram for schematically explaining another example of the packaging mode for irradiation with the gamma ray. In the mode shown in FIG. 2, the primary packaging article 3 accommodating the plurality of medical rubber products 1 is further accommodated in the secondary static charge prevention packaging article 5 and the tertiary static charge prevention packaging article 7. Each of the secondary static charge prevention packaging article 5 and the tertiary static charge prevention packaging article 7 is preferably sealed with the heat seal 9. As the primary packaging article 3, a packaging article having gas permeability is preferable. As the secondary static charge prevention packaging article 5 and the tertiary static charge prevention packaging article 7, packaging articles capable of sealing gas therein are preferable. The secondary packaging article 5 accommodating the primary packaging article 3 is filled with an inert gas. The filling with the inert gas makes it possible to set the oxygen concentration in each of the secondary packaging article 5 and the primary packaging article 3 to be not higher than 5%. Irradiation with the gamma ray can be performed with a plurality of the tertiary static charge prevention packaging articles 7 being accommodated in a quaternary packaging article (for example, a cardboard box).

At the time of irradiation with the gamma ray, irradiation with the gamma ray is preferably performed in a state

where the packaging article accommodating the plurality of medical rubber products is accommodated in, for example, an accommodation container made from an aluminum alloy.

The viable cell count (cfu: colony forming unit (the number of colonies that appear under incubation)), in a bioburden measurement test, of the sterilized medical rubber product according to the present invention is preferably 0.

Examples of the medical rubber product according to the present invention include: rubber plugs and sealing members of containers (for example, vials) for various medical preparations such as a liquid preparation, a powder preparation, and a freeze-dried preparation; slidable parts and sealing parts such as rubber plugs for vacuum blood collection tubes, and plunger stoppers and nozzle caps for pre-filled syringes; and the like.

EXAMPLES

Hereinafter, the present invention will be described in detail by means of examples, but the present invention is not limited to the following examples, and any of modifications and implementation modes made within the scope of the gist of the present invention is included in the scope of the present invention.

[Preparation of Medical Rubber Composition]

A rubber composition was prepared by blending components other than the crosslinking component (triazine-based crosslinking agent) among the components indicated in Table 1, kneading the resultant mixture with use of a 10-L pressurization-type sealed kneader at a filling rate of 75%, aging the kneaded product at room temperature, then, adding the crosslinking component to the kneaded product, and kneading the resultant mixture with use of an open roll.

[Table 1]

| Medical rubber composition | Blending amount (parts by mass) |
|---|---|
| Chlorinated isobutylene-isoprene rubber | 100 |
| Polyethylene | 5 |
| Triazine-based crosslinking agent | 1 |
| Talc | 50 |
| Hydrotalcite | 2 |
| Magnesium oxide | 2 |
| Carbon | 0.5 |
| Titanium oxide | 3 |
| Oil | 3 |

Details of the blending materials used are as follows.

Chlorinated isobutylene-isoprene rubber: 1066 (chlorine content: 1.26% by weight) manufactured by Exxon Mobil Corporation
Polyethylene: MIPELON XM-220 (degree of crystallinity: 69%) manufactured by Mitsui Chemicals, Inc.
Triazine derivative: ZISNET DB manufactured by SANKYO KASEI CO., LTD.
Talc: MISTRON Vapor manufactured by Imerys Specialties Japan Co., Ltd.
Hydrotalcite: ALCAMIZER 1 manufactured by Kyowa Chemical Industry Co., Ltd.
Magnesium oxide: MAGSARAT 150s manufactured by Kyowa Chemical Industry Co., Ltd.
Carbon black: DIABLACK G manufactured by Mitsubishi Chemical Holdings Corporation
Titanium oxide: KR-380 manufactured by Titan Kogyo Ltd.
Oil: PW380 manufactured by Idemitsu Kosan Co., Ltd.

[Manufacturing of Medical Rubber Plugs]

The aforementioned rubber composition was molded in the shape of a sheet, sandwiched between an upper mold and a lower mold, and press-molded in a vacuum at 180°C for 6 minutes, and a plurality of rubber plugs for vials for a freeze-dried injectable were continuously formed on the above one sheet. In each rubber plug, a flange had a diameter of 19.0 mm, the total height was 13.0 mm, a leg portion had a diameter of 7.20 mm, and a flange piercing

portion had a thickness of 2.5 mm. Thereafter, a silicone-based lubricating coat agent was applied on both surfaces of the above sheet. Then, rubber plugs were manufactured through an outer appearance inspection step, a stamping step, a cleaning step, a sterilizing step, and a drying step. Each of the manufactured rubber plugs was used in an eluting-substance test and a tack test. Conditions of sterilization with the gamma ray are indicated in Table 2.

[Table 2]

| Sterilization No. | | 1 | 2 | 3 | 4 | 5 | 6 | 7 |
|---|---|---|---|---|---|---|---|---|
| Conditions of irradiation with gamma ray | Packaging-article internal environment | Oxygen adsorber contained | Oxygen adsorber contained | Oxygen adsorber contained | Oxygen adsorber contained | Air | Air | Air |
| | Oxygen concentration in packaging article (%) | 0.1% | 0.1% | 0.1% | 0.1% | 21% | 21% | 21% |
| | Absorbed dose (kGy) | 15 | 25 | 50 | 100 | 50 | 100 | 200 |
| Analysis with FT-IR | (Ss/Si)×100 | 101 | 105 | 102 | 110 | 201 | 270 | 393 |
| | Ss=(As/Bs)×100 | 3.9 | 4.0 | 3.9 | 4.2 | 7.7 | 10.3 | 15.0 |
| | Si=(Ai/Bi)×100 | 3.8 | 3.8 | 3.8 | 3.8 | 3.8 | 3.8 | 3.8 |
| (1) Eluting-substance test | Overall evaluation | Pass | Pass | Pass | Pass | Pass | Pass | Not pass |
| (2) TOC eluting-substance test | Relative evaluation (after irradiation/before irradiation) | 93% | 100% | 119% | 129% | 398% | 783% | 1831% |
| | | A | A | A | B | C | C | C |
| (3) Tack test | Relative evaluation (after irradiation/before irradiation) | 95% | 96% | 102% | 118% | 355% | 803% | 1430% |
| | | A | A | A | A | C | C | C |
| (4) Viable cell count (cfu) | Test result (n=5) | 0 | 0 | 0 | 0 | 0 | 0 | 0 |
| Overall determination as to suitability for being ready-to-use | | Suitable | Suitable | Suitable | Suitable | Unsuitable | Unsuitable | Unsuitable |

[Evaluation Methods]

(1) Measurement with Fourier Transform Infrared Spectrophotometer (FT-IR)

A measurement sample was made by being cut out from the upper surface of each of the sterilized medical rubber plugs with use of a razor so as to have a thickness (about 1.0 mm) that allowed the measurement sample to be placed on a measurement device. The medical rubber plug was cut in the longitudinal direction through the center thereof in a plan view, and a measurement sample was obtained by being cut out from a center portion of the obtained cross section with use of the razor so as to have a thickness (for example, 0.5 mm to 2.0 mm) that allowed the measurement sample to be placed on the measurement device. Each of the measurement samples was placed on the measurement device, and a region, of a surface portion of the measurement sample, having an outer diameter of 1 mm was irradiated with light and was subjected to measurement.

FT-IR measurement was performed a total of 16 times at a wave number resolution of 4 $cm^{-1}$ by using Frontier with a MIRacle ATR unit (Ge) manufactured by PerkinElmer Co., Ltd.

FIG. 3 is an FT-IR chart of the surface portion of a medical rubber product having been subjected to sterilization No. 1. FIG. 4 is an FT-IR chart of the surface portion of a medical rubber product having been subjected to sterilization No. 7.

In each of the obtained infrared absorption spectra, an infrared absorption peak A having an absorption peak of absorbance at around a wave number of 1650 $cm^{-1}$, and an infrared absorption peak B having an absorption peak of absorbance at around a wave number of 1470 $cm^{-1}$, are present. For the infrared absorption peak A, a baseline was drawn between 1750 $cm^{-1}$ and 1590 $cm^{-1}$, and an area (As) delimited by the baseline was calculated. For the infrared absorption peak B, a baseline was drawn between 1510 $cm^{-1}$ and 1408 $cm^{-1}$, and an area (Bs) delimited by the baseline was calculated. Regarding the inside of the medical rubber plug as well, an area (Ai) of the infrared absorption peak A and an area (Bi) of the infrared absorption peak B were calculated in the same manner.

(2) Measurement of Melting Heat Quantity of Polyethylene

The melting heat quantity of the polyethylene was acquired from a primary test at elevated temperatures through differential scanning calorimetry (DSC).

DSC measurement condition: 20°C to 200°C, with the speed of temperature elevation being 10°C/min.

(3) Eluting-Substance Test

Measurement sample: in a state where each of the medical rubber plugs was put into a polyethylene bag (in either of the packaging modes shown in FIG. 1 and FIG. 2), the medical rubber plug was irradiated with the gamma ray in the corresponding packaging-article internal environment indicated in Table 2 so as to have the corresponding absorbed dose indicated in Table 2. Consequently, a rubber plug having been irradiated with the gamma ray was obtained.

The measurement sample was tested according to the method in "Extractable substances" described in "7.03 Test for Rubber Closure for Aqueous Infusions" of the 17th edition of the Japanese Pharmacopoeia. Conditions of passing the test were as follows.

Properties of test solution: colorless and clear
Ultraviolet transmissivity: a transmissivity being not lower than 99.0% at each of a wavelength of 430 nm and a wavelength of 650 nm with a layer length of 10 mm
Ultraviolet absorption spectrum: an absorbance being not higher than 0.20 at a wavelength of 220 nm to 350 nm
pH: the difference between the test solution and a blank test solution being not larger than 1.0
Zinc: the absorbance of a sample solution being not higher than the absorbance of a standard solution
Potassium permanganate reducing substance: not higher than 2.0 mL/100 mL (according to a standard in the Japanese Pharmacopoeia)
Post-evaporation residue: not larger than 2.0 mg

If any of these conditions was not satisfied, the measurement sample was evaluated as "Not pass". Meanwhile, if all of the conditions were satisfied, the measurement sample was evaluated as "Pass".

(4) TOC Test

Regarding the eluting liquid subjected to the eluting-substance test in "(3)", a total organic carbon value TOC was measured (through a non-purgeable organic carbon (NPOC) method).

Measurement analysis device: a Shimadzu total organic carbon analyzer TOC-$L_{CPH}$ (of a combustion oxidizing type)
Measurement analysis condition: a combustion tube temperature being 680 degrees with use of a high-sensitivity catalyst
Carrier gas: highly purified air at 150 mL/min.
Injection amount: 150 μL
Concentration of added acid: 1%
Aeration treatment time: 60 sec.

Eluting characteristics obtained before and after irradiation with the gamma ray were evaluated. The TOC obtained after irradiation with the gamma ray as a relative index was evaluated with the TOC obtained before irradiation with the gamma ray being regarded as 100%. A larger numeral means that the eluting performance has further deteriorated relative to the eluting performance obtained before irradiation with the gamma ray.

Evaluation criteria

A: not higher than 125% (equivalent to a pre-irradiation numeral)
B: higher than 125% and not higher than 150% (having slightly deteriorated relative to the pre-irradiation numeral)
C: higher than 150% (having deteriorated to a large extent, relative to the pre-irradiation numeral)

(5) Bioburden Measurement Test

As a bioburden measurement method, a method in which ultrasonic recovery and culture medium immersion are performed in combination was employed.

Operation procedure

a: Samples were transferred to test tubes (φ18 mm×180 mm) in which 10 mL of PTS recovery liquids (1% of peptone, 0.1% of Tween 80, and 0.85% of saline) had been respectively dispensed.
b: Ultrasonication was performed for 15 minutes.
c: Each of the recovery liquids was suctioned and filtered through a Milliflex membrane filter (MF).
d: The post-filtering Milliflex membrane filter (MF) was pasted on an SCDA (soybean-casein digest agar) plate culture medium.
e: The post-ultrasonication sample was transferred onto a deep petri dish and was immersed in an SCDA culture medium containing 0.002% of triphenyltetrazolium chloride.
f: The sample was cultured at 30°C to 35°C for seven days.
g: The culture medium after the culturing for seven days was observed, and the total of a viable cell count obtained through ultrasonic recovery and a viable cell count obtained through culture medium immersion was used as a viable cell count (cfu). The viable cell count (cfu) is the average value of viable cell counts with respect to five samples of each type.

(6) Tack Test

A tack test was performed as follows by using a desktop tester EZ-SX available from Shimadzu Corporation. That is, as shown in FIG. 5, a sample 13 was fixed to fixation jigs 15 on the lower side, a metal probe 17 on the upper side was pressed onto the sample 13, and the pressed state was maintained for 10 seconds after the pressure had reached a set value. Thereafter, the metal probe 17 was lifted upward, and a peak value of close contact force generated between the metal probe 17 and the sample 13 was used as a tack value. The measurement was performed 5 times on each sample. From among the measurement values obtained as a result, the maximum value and the minimum value were excluded, and the average value of the remaining three measurement values was calculated. The close contact force of the sample having been irradiated with the gamma ray was indicated as an index with the close contact force of the sample, which had not yet been irradiated with the gamma ray, being regarded as 100. A smaller index indicates a lower adhesiveness and a more favorable result.

Measurement conditions

Pressing speed: 0.5 mm/s
Pressing load: 1000 g of weight
Pressed-state maintaining time: 10 seconds
Pull-up speed: 10 mm/s
Ultimate pull-up distance: 3 mm
Diameter of probe: 10 mm

Evaluation criteria

A: not higher than 120% (equivalent to a pre-irradiation value)
B: higher than 120% and not higher than 150% (having slightly deteriorated relative to the pre-irradiation value)
C: higher than 150% (having deteriorated to a large extent, relative to the pre-irradiation value)

The results of the eluting-substance test, the TOC test, and the tack test are indicated together in Table 2.

Determination as to suitability for being ready-to-use was performed as follows.

If the result of the eluting-substance test was "Pass", the result of the TOC test was B or the more favorable evaluation result, the result of the tack test was B or the more favorable evaluation result, and the viable cell count (cfu) was zero, it was determined that suitability for being ready-to-use was attained. Meanwhile, if any one of the evaluation results was unsatisfactory, it was determined that suitability for being ready-to-use was not attained.

According to the results indicated in Table 2, non-eluting characteristics are maintained and less troubles occur in a medical product manufacturing process, in the case of a medical rubber product formed from an elastic material and sterilized by being irradiated with gamma ray or electron ray (beta ray), wherein, when measurement is performed on a surface portion of the elastic material through attenuated total reflection (ATR) by using a Fourier transform infrared spectrophotometer (FT-IR), if an area of an infrared absorption peak at around a wave number of 1650 $cm^{-1}$ in an infrared absorption spectrum obtained through the measurement is defined as As and an area of an infrared absorption peak at around a wave number of 1470 $cm^{-1}$ in the infrared absorption spectrum is defined as Bs, $Ss=(As/Bs)\times 100\leq 6$ is satisfied.

The present invention makes it possible to provide a medical rubber product in which non-eluting characteristics are maintained even after sterilization with gamma ray and with which less troubles occur in a medical product manufacturing process.

Preferable mode (1) of the present invention is directed to a medical rubber product formed from an elastic material and sterilized by being irradiated with gamma ray or electron ray (beta ray), wherein, when measurement is performed on a surface portion of the elastic material through attenuated total reflection (ATR) by using a Fourier transform infrared spectrophotometer (FT-IR), if an area of an infrared absorption peak at around a wave number of 1650 $cm^{-1}$ in an infrared absorption spectrum obtained through the measurement is defined as As and an area of an infrared absorption peak at around a wave number of 1470 $cm^{-1}$ in the infrared absorption spectrum is defined as Bs, $Ss=(As/Bs)\times 100\leq 6$ is satisfied.

Preferable mode (2) of the present invention is directed to the medical rubber product according to mode (1), wherein, when cut-out from a center portion of the medical rubber product is performed and measurement is performed on an infrared absorption spectrum of an exposed inside of the medical rubber product, if an area of an infrared absorption peak at around a wave number of 1650 $cm^{-1}$ in the infrared absorption spectrum is defined as Ai, an area of an infrared absorption peak at around a wave number of 1470 $cm^{-1}$ in the infrared absorption spectrum is defined as Bi, and $Si=(Ai/Bi)\times 100$ is satisfied, $P=(Ss/Si)\times 100\leq 150$ is satisfied on the inside.

Preferable mode (3) of the present invention is directed to the medical rubber product according to mode (1) or (2), wherein the elastic material is a cured product of a rubber composition containing: a (a) base polymer containing a halogenated isobutylene-isoprene rubber; and a triazine derivative as a (c) crosslinking agent.

Preferable mode (4) of the present invention is directed to the medical rubber product according to mode (3), wherein the halogenated isobutylene-isoprene rubber is at least one type of rubber selected from the group consisting of chlorinated isobutylene-isoprene rubbers, brominated isobutylene-isoprene rubbers, and brominated isobutylene-para-methylstyrene copolymer rubbers.

Preferable mode (5) of the present invention is directed to the medical rubber product according to mode (3) or (4), wherein the rubber composition further contains a (b) polyethylene.

Preferable mode (6) of the present invention is directed to the medical rubber product according to mode (5),

wherein the (b) polyethylene contains a polyethylene having a degree of crystallinity not higher than 70%.

Preferable mode (7) of the present invention is directed to the medical rubber product according to mode (5) or (6), wherein an amount of the (b) polyethylene contained per 100 parts by mass of the (a) base polymer is not smaller than 3 parts by mass and not larger than 30 parts by mass.

Preferable mode (8) of the present invention is directed to the medical rubber product according to any one of modes (1) to (7), wherein the elastic material has a JIS-A hardness of 30 to 70 degrees and a compression set not higher than 20%, the compression set being measured under conditions of 70°C, 22 hours, and 25% according to JIS K 6262.

Preferable mode (9) of the present invention is directed to the medical rubber product according to any one of modes (1) to (8), the medical rubber product being a rubber plug for a vial, a cap or a plunger stopper for a syringe, or a rubber plug for a vacuum blood collection tube.

## Claims

1. A medical rubber product (1) formed from an elastic material and sterilized by being irradiated with gamma ray or electron ray (beta ray), wherein, when measurement is performed on a surface portion of the elastic material through attenuated total reflection (ATR) by using a Fourier transform infrared spectrophotometer (FT-IR), if an area of an infrared absorption peak at around a wave number of 1650 $cm^{-1}$ in an infrared absorption spectrum obtained through the measurement is defined as As and an area of an infrared absorption peak at around a wave number of 1470 $cm^{-1}$ in the infrared absorption spectrum is defined as Bs, $Ss=(As/Bs)\times100\leq6$ is satisfied.

2. The medical rubber product (1) according to claim 1, wherein, when cut-out from a center portion of the medical rubber product (1) is performed and measurement is performed on an infrared absorption spectrum of an exposed inside of the medical rubber product (1), if an area of an infrared absorption peak at around a wave number of 1650 $cm^{-1}$ in the infrared absorption spectrum is defined as Ai, an area of an infrared absorption peak at around a wave number of 1470 $cm^{-1}$ in the infrared absorption spectrum is defined as Bi, and $Si=(Ai/Bi)\times100$ is satisfied, $P=(Ss/Si)\times100\leq150$ is satisfied on the inside.

3. The medical rubber product (1) according to claim 1 or 2, wherein the elastic material is a cured product of a rubber composition containing: a (a) base polymer containing a halogenated isobutylene-isoprene rubber; and a triazine derivative as a (c) crosslinking agent.

4. The medical rubber product (1) according to claim 3, wherein the halogenated isobutylene-isoprene rubber is at least one type of rubber selected from the group consisting of chlorinated isobutylene-isoprene rubbers, brominated isobutylene-isoprene rubbers, and brominated isobutylene-para-methylstyrene copolymer rubbers.

5. The medical rubber product (1) according to claim 3 or 4, wherein the rubber composition further contains a (b) polyethylene.

6. The medical rubber product (1) according to claim 5, wherein the (b) polyethylene contains a polyethylene having a degree of crystallinity not higher than 70%.

7. The medical rubber product (1) according to claim 5 or 6, wherein an amount of the (b) polyethylene contained per 100 parts by mass of the (a) base polymer is not smaller than 3 parts by mass and not larger than 30 parts by mass.

8. The medical rubber product (1) according to any one of claims 1 to 7, wherein the elastic material has a JIS-A hardness of 30 to 70 degrees and a compression set not higher than 20%, the compression set being measured under conditions of 70°C, 22 hours, and 25% according to JIS K 6262.

9. The medical rubber product (1) according to any one of claims 1 to 8, the medical rubber product (1) being a rubber plug for a vial, a cap or a plunger stopper for a syringe, or a rubber plug for a vacuum blood collection tube.

Fig. 1

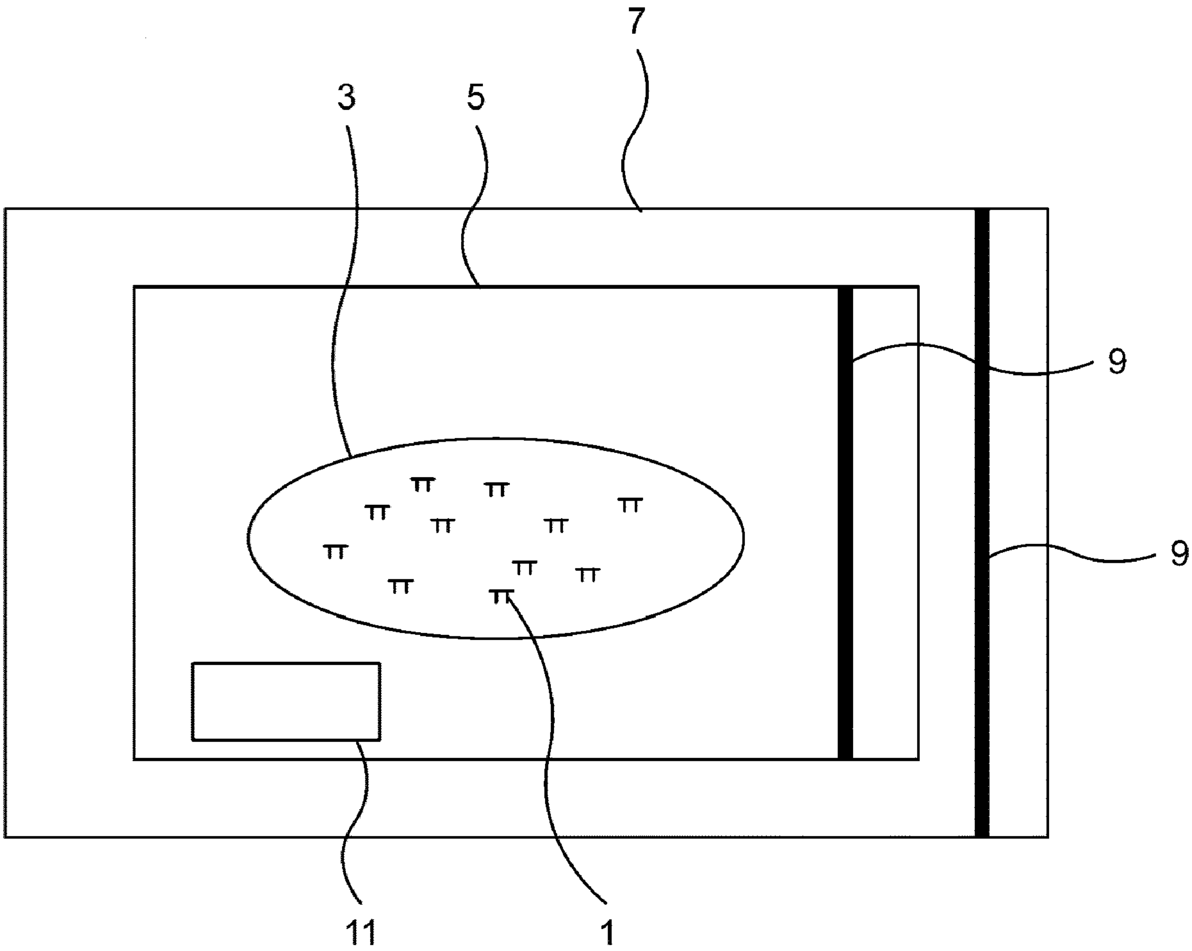

Fig. 2

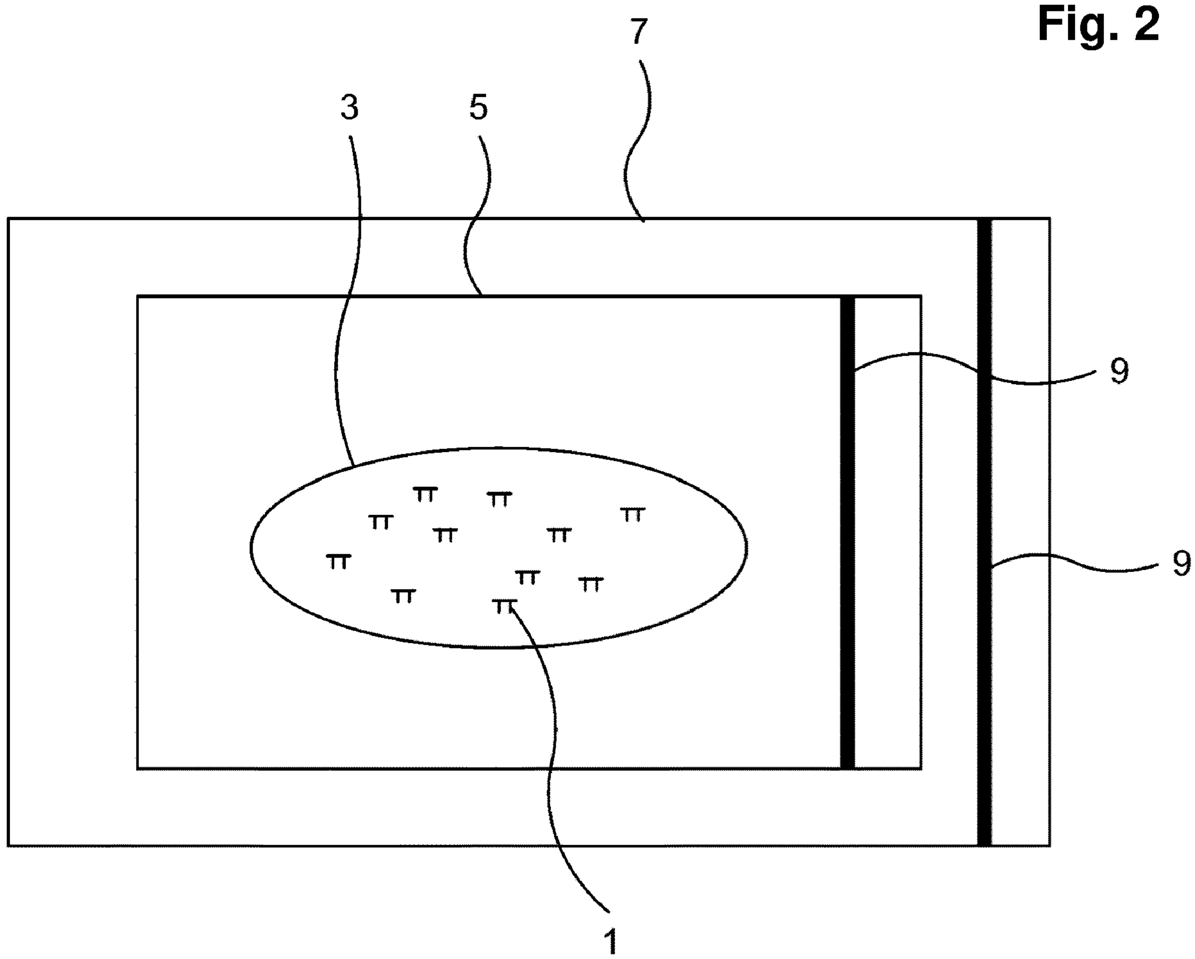

Fig. 3

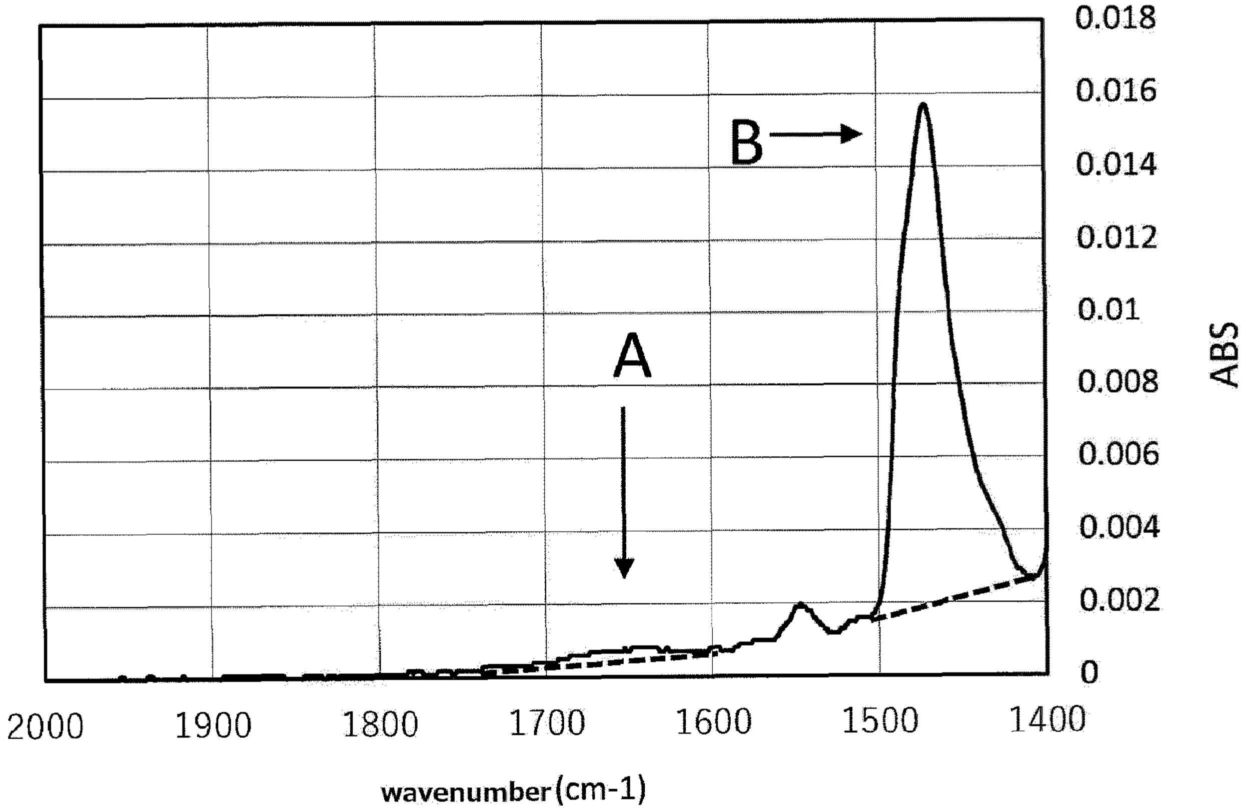
B
A
0.018
0.016
0.014
0.012
0.01
0.008
0.006
0.004
0.002
0
ABS
2000
1900
1800
1700
1600
1500
1400
wavenumber(cm-1)

**Fig. 4**

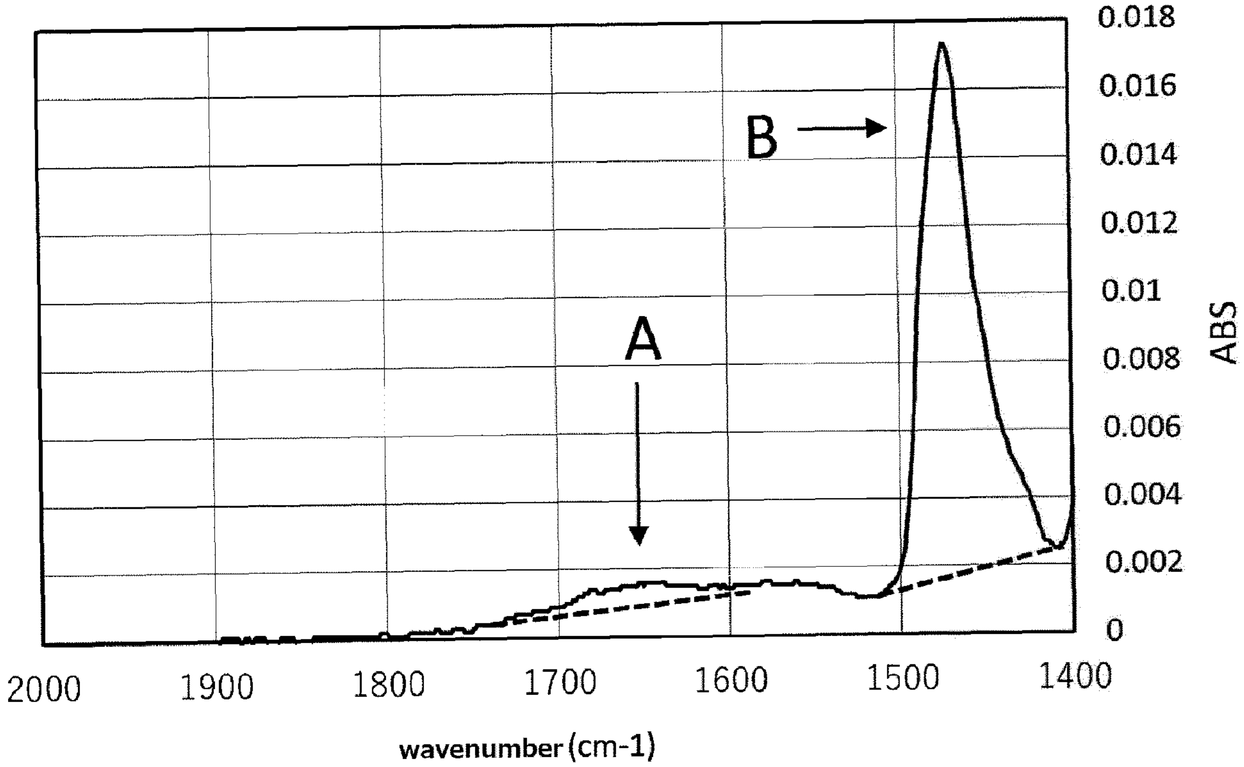
B
A
0.018
0.016
0.014
0.012
0.01
0.008
0.006
0.004
0.002
0
ABS
2000
1900
1800
1700
1600
1500
1400
wavenumber (cm-1)

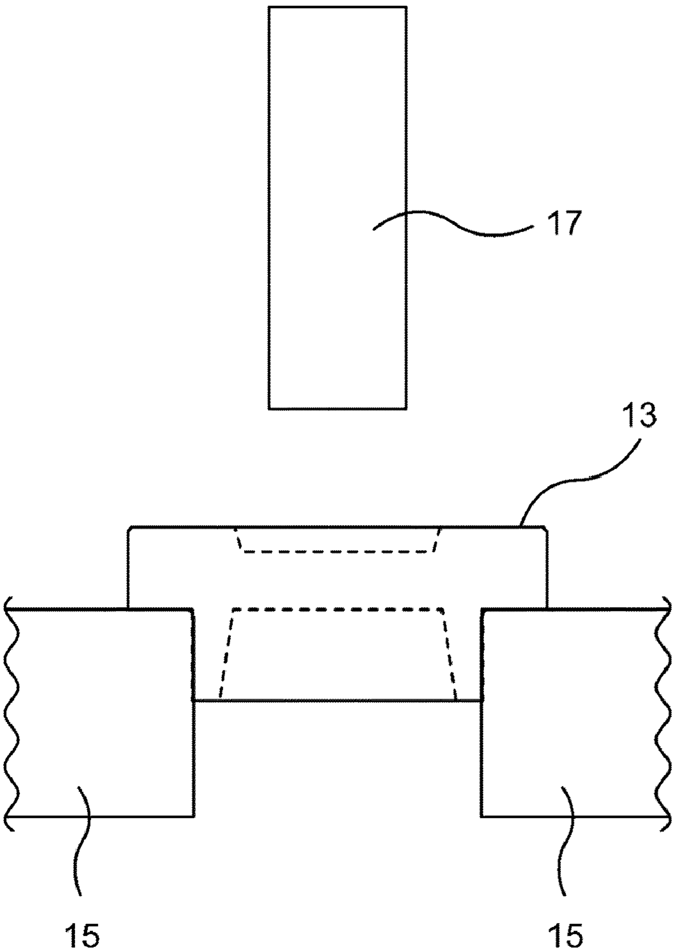


Fig. 5

Europäisches Patentamt
European Patent Office
Office européen des brevets

# EUROPEAN SEARCH REPORT

Application Number
EP 23 21 3586

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 227 126 A1 (DAIKYO SEIKO LTD [JP]) 31 July 2002 (2002-07-31)<br>* example 1 *<br>* claims 1-3, 9 *<br>----- | 1-5,7-9 | INV.<br>A61L2/08<br>A61L31/04 |
| X,P | EP 4 212 182 A2 (SUMITOMO RUBBER IND [JP]) 19 July 2023 (2023-07-19)<br>* page 5, paragraph 39 *<br>* claims 1, 2, 10 *<br>----- | 1-7,9 | |

TECHNICAL FIELDS SEARCHED (IPC)

A61L

The present search report has been drawn up for all claims

1

| Place of search | Date of completion of the search | Examiner |
|---|---|---|
| The Hague | 18 April 2024 | Heck, Georg |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO. EP 23 21 3586

This annex lists the patent family members relating to the patent documents cited in the above-mentioned European search report. The members are as contained in the European Patent Office EDP file on
The European Patent Office is in no way liable for these particulars which are merely given for the purpose of information.

18-04-2024

| Patent document cited in search report | Publication date | Patent family member(s) | Publication date |
|---|---|---|---|
| EP 1227126 A1 | 31-07-2002 | AT E333491 T1 | 15-08-2006 |
| | | DE 60213141 T2 | 02-08-2007 |
| | | DK 1227126 T3 | 04-12-2006 |
| | | EP 1227126 A1 | 31-07-2002 |
| | | ES 2267940 T3 | 16-03-2007 |
| | | US 2003100696 A1 | 29-05-2003 |
| EP 4212182 A2 | 19-07-2023 | CN 116327991 A | 27-06-2023 |
| | | EP 4212182 A2 | 19-07-2023 |
| | | JP 2023093154 A | 04-07-2023 |
| | | US 2023190970 A1 | 22-06-2023 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H10179690 A
- JP 2002301133 A
- JP 2017531604 W


### Non-patent literature cited in the description


- Test for Rubber Closure for Aqueous Infusions. Japanese Pharmacopoeia.